# EUROPÄISCHE PATENTSCHRIFT

(11) **EP 4 221 677 B1**
(45) Veröffentlichungstag und Bekanntmachung des Hinweises auf die Patenterteilung: **25.06.2025**
(21) Anmeldenummer: 21786474.3
(22) Anmeldetag: 01.10.2021
(51) Int. Cl.: A61K 8/44, A61K 31/198, A61K 31/045, A61K 31/164, A61K 31/522, A61K 47/10, A61K 9/00, A61K 9/08, A61K 31/355, A61P 17/14, A61Q 5/02, A61Q 5/12, A61Q 7/00

(54) **ZUSAMMENSETZUNG ZUR BEHANDLUNG VON HAAREN UND DER KOPFHAUT**
COMPOSITION FOR TREATING HAIR AND SCALP
COMPOSITION POUR LE TRAITEMENT DES CHEVEUX ET DU CUIR CHEVELU

(30) Priorität: 02.10.2020 DE 102020125876
(43) Veröffentlichungstag der Anmeldung: 09.08.2023
(73) Patentinhaber: Dr. Kurt Wolff GmbH & Co. KG, 33611 Bielefeld (DE)
(72) Erfinder: SCHULZE ZUR WIESCHE, Erik, 33611 Bielefeld (DE); BECKER, Maike, 33611 Bielefeld (DE); VÖLKER, Jörn Michael, 33611 Bielefeld (DE); KOCH, Nadine, 33611 Bielefeld (DE)
(74) Vertreter: Weickmann & Weickmann PartmbB
(86) Internationale Anmeldenummer: PCT/EP2021/077178
(87) Internationale Veröffentlichungsnummer: WO 2022/069744

(56) Entgegenhaltungen:
- DE-A1- 10 217 131
- DE-A1- 4 202 645
- US-A- 4 526 781
- US-A1- 2003 118 536
- JIA J ET AL: "Shampoo e.g. for softening and improving hair quality comprises modified pasty attapulgite clay, walnut liquid mixture, walnut powder mixture, honey, dodecyl dimethyl glycine, lauryl diethanolamine, citric acid and Nipagin methyl ester", WPI / 2017 CLARIVATE ANALYTICS,, vol. 2009, no. 12, 14 January 2009 (2009-01-14), XP002805080
- CONG L ET AL: "Anti-hair loss composition for shampoo, comprises adenosine, planktonic extract, caffeine, tocopherol acetate, polyethylene glycol-60 hydrogenated castor oil, glycyrrhizinate, acetyltetrapeptide-3, arginine and menthol", WPI / 2017 CLARIVATE ANALYTICS,, vol. 2020, no. 12, 17 January 2020 (2020-01-17), XP002805196
- CHEN J: "Hair conditioning shampoo contains tea oil, beeswax, Angelica, black sesame seeds, oriental arborvitae leaf, guar gum, lauryl dimethyl glycine, ammonium dodecyl sulfate, peppermint oil, black hair brightening agent, and alkyl glucoside", WPI / 2017 CLARIVATE ANALYTICS,, vol. 2017, no. 8, 23 November 2016 (2016-11-23), XP002805079
- DATABASE WPI Week 201708, Derwent World Patents Index; AN 2016-74074T, XP002805079
- DATABASE WPI Week 200912, Derwent World Patents Index; AN 2009-E24549, XP002805080
- DATABASE WPI Week 202001, 20 December 2021 Derwent World Patents Index; AN 2020-085211, XP002805196

## Beschreibung

Die vorliegende Erfindung betrifft eine Zusammensetzung, die Dimethylglycin und/oder ein Salz von Dimethylglycin enthält. Zudem betrifft die vorliegende Erfindung die (kosmetische und/oder medizinische) Verwendung dieser Zusammensetzung zur Behandlung von Haaren und der Kopfhaut.

Das menschliche Haar hat seine Bedeutung als Schutzfunktion für den Körper mittlerweile zu einem großen Teil verloren. Allerdings hat gesundes Haar weltweit eine große kulturelle Bedeutung für Frauen und Männer und wird häufig als Zeichen von Wohlstand und Gesundheit angesehen. Folglich beeinflusst beispielsweise durch Haarausfall bedingtes schütteres Haar oft die Lebensqualität auf negative Weise. Ein durch Hormone wie Androgene hervorgerufener Haarausfall wird als androgenetische Alopezie (Alopecia androgenetica oder auch AGA) bezeichnet und gilt insbesondere bei Männern aber ebenfalls auch bei Frauen als die häufigste Ursache für Haarausfall. Hierbei kann zwischen männlichen und weiblichen Haarausfallmustern unterschieden werden.

Das Haarwachstum und das Nachwachstum von Haaren hängt von einer ausreichenden Nährstoffversorgung der Haarfollikel ab, welche über das Blut bereitgestellt wird. Eine verringerte Durchblutung (Mikrozirkulation) der Kopfhaut kann somit Haarausfall begünstigen oder gar verursachen. Bei glatzköpfigen Männern ist beispielsweise die Durchblutung der Vertex-Region der Kopfhaut signifikant geringer als bei Männern mit normaler Kopfhautbehaarung. Diese signifikant verringerte Mikrozirkulation kann also eine Erklärung für den Haarausfall sowie das ausbleibende Nachwachsen der Haare (Übergang von Telogen zu Anagen) beispielsweise bei der androgenetischen Alopezie sein. Insgesamt kann Haarausfall also als eines der ersten klinischen Anzeichen für einen verminderten Blutdurchfluss der peripheren Gefäße angesehen werden.

Bis heute gibt es nur wenige zugelassene pharmazeutische Behandlungsmöglichkeiten für Haarausfall, wie androgenetische Alopezie, und die bereits vorhandenen Behandlungsmöglichkeiten sind entweder nicht ausreichend wirksam oder gehen mit unangenehmen Nebenwirkungen einher. Ähnlich unerfreulich sieht es beispielsweise bei Behandlungsmöglichkeiten für den akut einsetzenden entzündlich bedingten kreisrunden Haarausfall, der auch als Alopecia areata oder kurz AA bezeichnet wird, und für durch andere Ursachen bedingten Haarausfall aus.

DE 10 217 131 A1 offenbart eine topisch applizierbare Zusammensetzung zur Behandlung der Haare oder Nägel, die einen Wirkstoff und ein Trägersystem aufweist, wobei das Trägersystem aus der Gruppe ausgewählt ist, die Wasser, ein organisches Lösungsmittel, einen Emulgator, einen Gelbildner, eine vesikelausbildende Substanz und/oder eine Pudergrundsubstanz umfasst.

US 4,526,78 A1 offenbart DMG-Derivate mit Betainstruktur in HaarpflegeZusammensetzungen ("hydrogenated talau betaine").

Damit besteht weiterhin ein Bedarf an verbesserten Behandlungsmethoden, insbesondere ein Bedarf an Zusammensetzungen, welche topisch gegen Haarausfall eingesetzt werden können, wobei diese Zusammensetzungen keine oder nur vernachlässigbare Nebenwirkungen zeigen sollen. Insbesondere besteht ein Bedarf an Zusammensetzungen, welche pharmazeutischer Natur sind und keine Nebenwirkungen zeigen oder welche kosmetischer Natur sind.

Ausgehend hiervon war es die Aufgabe der vorliegenden Erfindung, eine gut verträgliche Zusammensetzung zur Behandlung von Haaren und der Kopfhaut, insbesondere zur Prävention und/oder Behandlung von Haarausfall, bereitzustellen. Weiterhin sollte sich diese Zusammensetzung topisch anwenden lassen und die Nachteile der aus dem Stand der Technik bekannten Zusammensetzungen überwinden.

Diese Aufgabe wurde überraschend durch die Verwendung gemäß Anspruch 1 gelöst. Bevorzugte Ausführungsformen gehen aus den abhängigen Ansprüchen hervor.

Erfindungsgemäß wird die Aufgabe durch die Verwendung einer Zusammensetzung gelöst, welche Dimethylglycin und/oder ein Salz von Dimethylglycin umfasst, wobei das Salz von Dimethylglycin ein Alkali-, Erdalkali- oder Ammoniumsalz von Dimethylglycin oder das Salz einer anorganischen und/oder organischen Säure mit Dimethylglycin ist.

Es hat sich überraschend gezeigt, dass die erfindungsgemäße Verwendung von Dimethylglycin und/oder einem Salz von Dimethylglycin wie beansprucht eine ausgezeichnete Wirksamkeit in der Behandlung von Haarausfall, wobei der Haarausfall ausgewählt ist aus Alopecia areata (kreisrundem Haarausfall), Alopecia androgenetica (erblich bedingter Haarausfall) bei Frauen und Männern, diffuser Alopezie (diffusem Haarausfall), altersbedingtem Haarausfall (senescent Alopecia) und durch Chemotherapie-bedingtem Haarausfall, aufweist. Die verwendete Zusammensetzung aktiviert die Kopfhaut und verbessert die Nährstoff- und Sauerstoffversorgung der Kopfhaut und der Haarwurzel deutlich. Zudem ist sie auf der Kopfhaut medizinisch und kosmetisch äußerst verträglich.

Dimethylglycin (N,N-Dimethylglycin) kommt in Pflanzen, Tieren und dem Menschen vor, wobei es im Menschen nur in sehr geringen Mengen gebildet wird. Es entsteht bei einer mehrstufigen Biosynthese von Glycin aus Cholin als Zwischenprodukt durch Transaminierung von Betain mit Betain-Homocystein-Methylase.

N,N-Dimethylglycin, auch (Dimethylamino)essigsäure, wird durch die folgende chemische Formel 1 dargestellt:

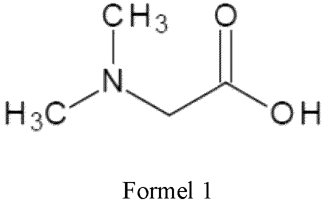

Erfindungsgemäß ist nicht nur der Einsatz von Dimethylglycin, sondern auch der von dessen Salzen, Solvaten und Hydraten. Dabei handelt es sich bevorzugt um pharmazeutisch bzw. kosmetisch annehmbare Salze von Dimethylglycin. Das Salz ist besonders bevorzugt ein wasserlösliches Salz mit einer Löslichkeit in Wasser von mindestens 10 g/l bei 20°C.

Das Salz von Dimethylglycin ist erfindungsgemäß ein Alkali-, Erdalkali- oder Ammoniumsalz von Dimethylglycin oder das Salz einer anorganischen und/oder organischen Säure mit Dimethylglycin.

Beispiele sind Natrium-, Kalium-, Calcium-, Magnesium- und Ammoniumsalze. Bei den Ammoniumsalzen trägt das Ammoniumkation eine bis vier Alkylgruppen mit jeweils unabhängig voneinander 1 bis 4 Kohlenstoffatomen. Bevorzugt sind das Natrium- und Kaliumsalz von Dimethylglycin, insbesondere das Natriumsalz von Dimethylglycin, nämlich Natrium-*N,N-*dimethylglycinat.

Beispiele für Salze von Dimethylglycin mit einer anorganischen Säure sind das Hydrochlorid, Hydrobromid, Hydroiodid, Hydrogensulfat, Sulfat, Hydrogensulfit, Sulfit, Hydrogencarbonat, Carbonat, Monophosphat, Diphosphat und Triphosphat von Dimethylglycin sowie Mischungen daraus. Insbesondere bevorzugt ist das Hydrochlorid von Dimethylglycin.

Beispiele für Salze von Dimethylglycin mit einer organischen Säure sind das Acetat, Laktat, Citrat, Succinat, Fumarat, Maleat und Benzoat von Dimethylglycin sowie Mischungen daraus.

Es wird angenommen, dass Dimethylglycin und/oder ein Salz von Dimethylglycin wie beansprucht erfindungsgemäß die Zellaktivität und den Sauerstoffumsatz in den Keratinocyten verbessert und damit auch die Zellaktivität in der Kopfhaut und in den Haarfollikeln fördert. Es erzielt so erfindungsgemäß eine deutliche haarwurzel- und kopfhautstärkende Wirkung, insbesondere bei der Behandlung von Haarausfall, wie dem erblich bedingten und altersbedingten Haarausfall.

Der pH-Wert der topischen Zusammensetzung beträgt bevorzugt 3,0 bis 5,9, vorzugsweise 3,5 bis 5,4 und besonders bevorzugt 4,0 bis 5,0 (gemessen bei 21 °C mittels pH-Meter, Mettler-Toledo SevenCompact S220). In diesem Bereich sind die erfindungsgemäß verwendeten Zusammensetzungen nicht nur chemisch, physikalisch und mikrobiologisch besonders stabil, sondern auch medizinisch und kosmetisch äußerst verträglich auf Kopfhaut und Haaren. Besonders geeignete pH-Werte für die erfindungsgemäß verwendete Zusammensetzung sind: 3,5; 3,6; 3,7; 3,8; 3,9; 4,0; 4,1; 4,2; 4,3; 4,4; 4,5; 4,6; 4,7; 4,8; 4,9; 5,0; 5,1; 5,2; 5,3; und 5,4. Der pH-Wert der Zusammensetzung wird bevorzugt eingestellt mithilfe eines oder mehrerer pH Modifizierungsmittel. Geeignete pH-Modifizierungsmittel können Säuren, Basen und/oder Puffersysteme sein, um den pH-Wert der Zusammensetzung zu stabilisieren oder zu beeinflussen. Typische pH-Modifizierungsmittel gemäß der vorliegenden Erfindung sind Adipin-, Zitronen-, Äpfel-, Bernstein-, Wein-, Ascorbin-, Phosphor-, Milch- und Fumarsäure sowie die entsprechenden Salze, sowie Natriumalginat, Polyacrylsäure, Natriumcarbonat und Natriumbicarbonat. Im Zusammenhang mit pH-Modifizierungsmitteln bezieht sich der Begriff Salz auf Alkalimetallsalze oder Erdalkalimetallsalze, sofern nichts anderes angegeben ist.

Die erfindungsgemäß verwendete Zusammensetzung enthält Dimethylglycin und/oder ein Salz von Dimethylglycin wie beansprucht bevorzugt in einem Anteil von 0,00001 Gew.-% bis 25,0 Gew.-%, bezogen auf das Gesamtgewicht der Zusammensetzung. In einer bevorzugten Ausführungsform enthält die erfindungsgemäße Zusammensetzung Dimethylglycin und/oder ein Salz von Dimethylglycin in einem Anteil von 0,001 Gew.-% bis 10,0 Gew.-%, bevorzugter von 0,01 Gew.-% bis 8,0 Gew.-%, mehr bevorzugt von 0,1 Gew.-% bis 6,0 Gew.-%, noch mehr bevorzugt von 0,3 Gew.-% bis 5,0 Gew.-%, insbesondere von 0,5 Gew.-% bis 3,0 Gew.-%, jeweils bezogen das Gesamtgewicht der Zusammensetzung. In einer bevorzugten Ausführungsform der Erfindung kann die erfindungsgemäße Zusammensetzung 0,1 Gew.%, 0,2 Gew.%, 0,3 Gew.%, 0,4 Gew.%, 0,5 Gew.%, 0,6 Gew.%, 0,7 Gew.%, 0,8 Gew.%, 0,9 Gew.%, 1,0 Gew.%, 1,1 Gew.%, 1,2 Gew.%, 1,3 Gew.%, 1,4 Gew.%, 1,5 Gew.%, 2,0 Gew.% oder 2,5 Gew.% Dimethylglycin und/oder ein Salz von Dimethylglycin wie beansprucht enthalten, jeweils bezogen auf das Gesamtgewicht der Zusammensetzung. Bevorzugt enthält die erfindungsgemäße Zusammensetzung Dimethylglycin und/oder ein Salz von Dimethylglycin wie beansprucht als chemischen Reinstoff, einschließlich der jeweiligen Solvate und Hydrate (z.B. des Dihydrats von Natriumdimethylglycinat), da so die Reinheit der Zusammensetzung erhöht und das Auftreten von unerwünschten Nebenwirkungen vermindert werden kann.

In einer bevorzugten Ausführungsform enthält die erfindungsgemäß verwendete Zusammensetzung mindestens einen weiteren Wirkstoff, wobei der mindestens eine weitere Wirkstoff ausgewählt ist aus Koffein, Menthol, Biotin, Zink PCA, Niacinamid, Panthenol, Ectoin, Ubichinon-10, Taurin, Pantolacton, Echinacea, Carnitin, Tocopherylacetat und Kombinationen davon. Die Kombination von Dimethylglycin bzw. den Salzen von Dimethylglycin mit Koffein ist erfindungsgemäß besonders bevorzugt.

Der IUPAC-Name von Koffein ist 1,3,7-Trimethyl-3,7-dihydro-1H-purin-2,6-dion. Alternativ wird Koffein auch als 1,3,7-Trimethylxanthin bezeichnet. Koffein wird durch die folgende chemische Formel 2 dargestellt.

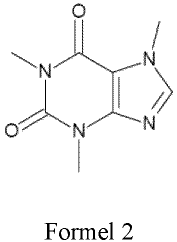

Koffein ist ein zur Klasse der Methylxanthine gehörendes Methylxanthinalkaloid. Es ist eine bittere kristalline Substanz, kann als Purinderivat betrachtet werden und ist chemisch mit den Adenin- und Guaninbasen der Desoxyribonukleinsäuren und der Ribonukleinsäure verwandt.

So zeigt die erfindungsgemäß verwendete Wirkstoffkombination von Dimethylglycin bzw. einem Salz von Dimethylglycin wie beansprucht mit Koffein eine verbesserte Wirksamkeit in der Behandlung von Haarausfall, insbesondere von erblich bedingtem und altersbedingtem Haarausfall, als Koffein oder Dimethylglycin und/oder ein Salz von Dimethylglycin wie beansprucht alleine. Die Wirkstoffkombination steigert dabei die Mikrozirkulation in der Kopfhaut in unerwartetem Maße und verbessert die Nährstoff- und Sauerstoffversorgung der Haarwurzel deutlich.

In einer bevorzugten Ausführungsform enthält die erfindungsgemäß verwendete Zusammensetzung Koffein in einem Anteil von 0,001 Gew.-% bis 3,0 Gew.-%, mehr bevorzugt 0,005 Gew.-% bis 2,50 Gew.-%, noch mehr bevorzugt von 0,01 Gew.-% bis 2,0 Gew.-%, insbesondere von 0,1 Gew.-% bis 1,5 Gew.-%, jeweils bezogen auf das Gesamtgewicht der Zusammensetzung. In einer bevorzugten Ausführungsform der Erfindung kann die Zusammensetzung 0,01 Gew.%, 0,05 Gew.%, 0,1 Gew.%, 0,2 Gew.% 0,3 Gew.%, 0,4 Gew.%, 0,5 Gew.%, 0,6 Gew.%, 0,7 Gew.%, 0,8 Gew.%, 0,9 Gew.%, 1,0 Gew.%, 1,1 Gew.%, 1,2 Gew.%, 1,3 Gew.%, 1,4 Gew.%, oder 1,5 Gew.%, Koffein enthalten, jeweils bezogen auf das Gesamtgewicht der Zusammensetzung.

Das Gewichtsverhältnis zwischen Dimethylglycin und/oder einem Salz von Dimethylglycin wie beansprucht sowie Koffein liegt vorzugsweise in einem Bereich von 10 : 1 bis 1 : 10, bevorzugt von 6 : 1 bis 1 : 6, mehr bevorzugt von 4 : 1 bis 1 : 4, besonders bevorzugt von 2 : 1 bis 1 : 2. In einer bevorzugten Ausführungsform der Erfindung ist das Gewichtsverhältnis zwischen Dimethylglycin und/oder einem Salz von Dimethylglycin wie beansprucht sowie Koffein 0,5; 0,6; 0,7; 0,8; 0,9; 1,0; 1,1; 1,2; 1,3; 1,4; 1,5; 1,6; 1,7; 1,8; 1,9 oder 2,0.

Erfindungsgemäß bevorzugt ist weiterhin die Kombination von Dimethylglycin bzw. einem der Salze von Dimethylglycin wie beansprucht, entweder alleine oder besonders bevorzugt zusammen mit Koffein, und einem oder mehreren der folgenden Wirkstoffe.

Alternativ enthält die erfindungsgemäß verwendete topische Zusammensetzung gerade kein Koffein. Diese Ausführungsform wird auch als (vollständig) koffeinfreie topische Zusammensetzung bzw. als erfindungsgemäß verwendete topische Zusammensetzung ohne Koffein bezeichnet, für die abgesehen davon alle weiteren hierein beschriebenen bevorzugten Merkmale der Erfindung gelten.

Menthol ist ein monocyclischer Monoterpenalkohol und kann der erfindungsgemäß verwendeten Zusammensetzung als die Durchblutung stimulierender Wirkstoff zugesetzt werden. Zudem kann Menthol eine erfrischende sensorische Stimulanz der Kopfhaut bewirken.

Biotin, welches auch als Vitamin B7 oder Vitamin H bezeichnet wird, ist ein wasserlösliches Vitamin aus dem B-Komplex. Biotin kann erfindungsgemäß Haarausfall weiter vermindern und die Kopfhaut stärken.

Zink PCA ist das Zinksalz des L-Pyrrolidon-Carboxylats und kann als Substanz mit antimikrobieller Wirkung der erfindungsgemäß verwendeten Zusammensetzung zugesetzt werden.

Niacinamid (auch Nicotinamid) ist das Amid der Nicotinsäure und wird auch als Vitamin B3 bezeichnet. Neben anderen Eigenschaften wie beispielweise einer Verringerung von oxidativem Stress hat das Niacinamid erfindungsgemäß eine das Haarwachstum stimulierende Wirkung.

Panthenol ist ein Provitamin, welches im Körper zu Pantothensäure (Vitamin B5) umgewandelt wird. Letzteres ist Teil des Coenzyms A und damit für den Hautstoffwechsel wichtig. Bei Einwirkung von Panthenol wird erfindungsgemäß die Hautelastizität und Feuchtigkeit weiter verbessert. Daneben werden Juckreiz und Entzündungen gelindert und die Wundheilung gefördert.

Ectoin ist eine zyklische Aminosäure und liegt in wässriger Lösung als mesomeriestabilisiertes Zwitterion vor. Ectoin wirkt befeuchtend und stabilisiert erfindungsgemäß die natürliche Struktur von Haaren weiter. Zudem hat sich gezeigt, dass Ectoin vor UV-Strahlung schützt und bei der Behandlung entzündlicher Krankheiten hilfreich sein kann.

Ubichinon-10 (Q10 oder Coenzym Q10) ist ein Chinon-Derivat. Das zum Ubichinon-Pool gehörende Q10 gilt als Antioxidans und wirkt erfindungsgemäß stabilisierend auf die Kopfhaut und Haare und insbesondere die Haarwurzel.

Taurin oder 2-Aminoethansulfonsäure wirkt erfindungsgemäß als Antioxidans ebenfalls weiter stabilisierend auf die Kopfhaut und Haare und insbesondere die Haarwurzel.

Pantolacton entstammt der Gruppe der substituierten Lactone und regt erfindungsgemäß die Wachstumsfaktoren der Haarwurzeln weiter an.

Echinacea wirkt erfindungsgemäß beruhigend auf die Kopfhaut und lindert auftretenden Juckreiz sowie Spannungen. Zudem kann Echinacea die Blutzirkulation der Kopfhaut anregen und so die Haarfollikel mit sauerstoff- und nährstoffreichem Blut versorgen, was sich weiter stabilisierend auf die Haare und insbesondere die Haarwurzel auswirkt.

Carnitin hemmt vermutlich die Bildung von haarwuchsreduzierenden Faktoren und wirkt erfindungsgemäß auf diese Weise weiter gegen Haarausfall.

Tocopherylacetat zeigt antioxidative Eigenschaften und wirkt erfindungsgemäß weiter stabilisierend auf die Kopfhaut und die Haare und insbesondere die Haarwurzel.

In einer bevorzugten Ausführungsform der Erfindung enthält die erfindungsgemäß verwendete Zusammensetzung mindestens einen weiteren Wirkstoff ausgewählt aus Menthol, Biotin, Zink PCA, Niacinamid, Panthenol, Ectoin, Ubichinon, Taurin, Pantolacton, Echinacea, Carnitin, Tocopherylacetat und Kombinationen davon jeweils in einem Anteil von 0,001 Gew.-% bis 10,0 Gew.-%, mehr bevorzugt 0,005 Gew.-% bis 7,50 Gew.-%, noch mehr bevorzugt von 0,01 Gew.-% bis 5,0 Gew.-%, insbesondere von 0,1 Gew.-% bis 3,0 Gew.-%, bezogen auf das Gesamtgewicht der Zusammensetzung.

Darüber hinaus kann die erfindungsgemäße Zusammensetzung wasserbasiert sein. Das bedeutet, dass sie beispielsweise 45,0 bis 85,0 Gew.-% Wasser enthält.

Bevorzugt weist die Zusammensetzung (insbesondere als Shampoo oder andere halbfeste Formulierung) eine Viskosität von 800 bis 6000 mPa·s, besonders bevorzugt von 1000 bis 5700 mPa·s und ganz besonders bevorzugt von 2500 bis 5500 mPa·s auf, jeweils gemessen gemäß DIN 53019-1:2008-09 mit dem Rheometer Haake RheoStress1 (ThermoFisher Scientific) bei 20°C und einer Schergeschwindigkeit von 10/s in Platte-Platte Geometrie (Drehkörper PP60 Ti).

Alternativ weist die Zusammensetzung (insbesondere als Tonikum/Haarwasser) eine Viskosität von 0,1 bis 1000 mPa·s, besonders bevorzugt von 0,5 bis 500 mPa·s und ganz besonders bevorzugt von 1 bis 100 mPa·s auf, jeweils gemessen gemäß DIN 53019-1:2008-09 mit dem Rheometer Haake RheoStress1 (ThermoFisher Scientific) bei 20°C und einer Schergeschwindigkeit von 10/s in Platte-Platte Geometrie (Drehkörper PP60 Ti). Ganz besonders bevorzugt ist die topische Zusammensetzung bei 20°C (Raum- bzw. Umgebungstemperatur) eine niederviskose (dünnflüssige) Zusammensetzung ähnlich der Erscheinung von Wasser.

In einer Ausführungsform, insbesondere als Shampoo oder andere halbfeste Formulierung, umfasst die Zusammensetzung ein Tensid. Das Tensid kann ein anionisches, nichtionisches, kationisches oder zwitterionisches Tensid sein. Bevorzugt ist das Tensid ein anionisches oder nichtionisches Tensid, insbesondere ein möglichst mildes (d.h. besonders hautverträgliches) anionisches oder nichtionisches Tensid. Nichtionische Tenside werden dabei erfindungsgemäß insbesondere wegen ihrer sehr guten Emulgationseigenschaften sowie ihrer ausgezeichneten Hautpflegeeigenschaften eingesetzt. Anionische Tenside sind bevorzugt, weil sie eine besonders hohe Reinigungsleistung aufweisen. Daher eignen sie sich besonders gut in Reinigungszusammensetzungen wie Shampoos. Kationische Tenside besitzen hervorragende Haarpflegeeigenschaften und werden erfindungsgemäß insbesondere in Haarpflegezusammensetzungen eingesetzt wie in Conditionern, Shampoos und Kuren.

Die erfindungsgemäß verwendete Zusammensetzung enthält Tenside vorzugsweise in einer Menge von 2 bis 40 Gew.%, insbesondere von 5 bis 30 Gew.%, bevorzugt von 7 bis 20 Gew.%, besonders bevorzugt von 10 bis 17 Gew.%, jeweils bezogen auf das Gesamtgewicht der Zusammensetzung. Geeignete Mengen an Tensid lauten: 8 Gew.%; 9 Gew.%; 10 Gew.%; 11 Gew.%; 12 Gew.%; 13 Gew.%; 14 Gew.%; 15 Gew.%; 16 Gew.%; 17 Gew.%; 18 Gew.%; 19 Gew.%; 20 Gew.%; 21 Gew.%; 22 Gew.%; 23 Gew.%; 24 Gew.%; 25 Gew.%, jeweils bezogen auf das Gesamtgewicht der Zusammensetzung. Besonders bevorzugt enthält die topische Zusammensetzung der Erfindung ein oder mehrere anionische Tenside in einer Menge von 0,1 bis 20 Gew.%, bevorzugt von 1 bis 17 Gew.% und besonders bevorzugt von 5 bis 15 Gew.%, jeweils bezogen auf das Gesamtgewicht der Zusammensetzung. Geeignete Mengen an anionischem Tensid lauten: 1 Gew.%; 2 Gew.%; 3 Gew.%; 4 Gew.%; 5 Gew.%; 6 Gew.%; 7 Gew.%; 8 Gew.%; 9 Gew.%; 10 Gew.%; 11 Gew.%; 12 Gew.%; 13 Gew.%; 14 Gew.%; 15 Gew.%; 16 Gew.%; 17 Gew.%; 18 Gew.%, 19 Gew.%, 20 Gew.%, jeweils bezogen auf das Gesamtgewicht der Zusammensetzung. In diesen Mengen weisen die Tenside eine besonders hohe Reinigungsleistung auf und sind äußerst verträglich für Kopfhaut und Haare.

Die Tenside der vorliegenden Erfindung sind u.a. in dem Buch *"*Surfactants and interfacial phenomena", von Milton Rosen und Joy Kunjappu, John Wiley & Sons, Inc.-Verlag, 2012, 4. Auflage beschrieben.

In einer bevorzugten Ausführungsform ist das Tensid ein anionisches Tensid ausgewählt aus Alkylsulfonaten, Alkylsulfaten, Alkylethersulfaten, Alkylphosphaten, Alkylsarkosinaten, Alkyltauraten, Aminosäure-Tensiden und Mischungen davon. Besonders bevorzugt ist das Tensid ausgewählt aus Alkylsulfaten, Alkylsarkosinaten, Alkyltauraten, Alkylglutamat, wie Natriumcocoylglutamat/Dinatriumcocoylglutamat, Alkylgycinat, Alkylalaninat, wie Natriumcocoylalaninat und Mischungen davon. Ebenso bevorzugt wegen ihrer Reinigungsleistung sind Fettalkoholpolyglycerolethersulfate, Monoglyceridsulfate, Mono- und/oder Dialkylsulfosuccinate, Fettsäureisethionate, und □-Olefinsulfonate.

Alkylsulfate haben die generische Formel ROSO3M , Alkylsarkosinate haben die generische Formel RC(O)N(CH3)CH2CO2M, und Alkyltaurate haben die generische Formel RC(O)N(CH3)CH2CH2SO3M, wobei R jeweils ein C4-C26 Alkyl oder C4-C26 Alkenyl ist und M ein wasserlösliches Kation wie z.B. Ammonium, Natrium oder Kalium darstellt. Vorzugsweise ist M ein Natriumkation. Bevorzugt ist R ein C₁₂-C₁₆ Alkyl oder ein C₁₂-C₁₈ Alkyl.

In einer Ausführungsform ist das Tensid ein nichtionisches Tensid. Nicht-einschränkende Beispiele für ein nichtionisches Tensid umfassen Glycerinfettsäureester, Polyoxyethylenether eines oder mehrerer Fettalkohole, alkoxylierte Fettsäurealkylester, Polyglycerinether von Fettalkoholen, Polyglycerinester von Fettsäuren, Polyethylenglykol- und/oder Polypropylenglykolether, Fettsäureamide, Alkylphenolpolyglycolether, Aminoxide und Alkylpolyglucoside.

In einer Ausführungsform ist das Tensid ausgewählt aus der Gruppe der Glycerinfettsäureester, Polyoxyethylenether eines oder mehrerer Fettalkohole, Polyglycerinether von Fettalkoholen, Polyglycerinester von Fettsäuren und deren Mischungen.

In der vorliegenden Erfindung bezieht sich der Begriff "Glycerinfettsäureester" auf einen Glycerinmono- oder Glycerindifettsäureester. Glycerindifettsäureester weisen die Formel R³-COO-(CH₂CH(OH)CH₂)-OOR⁴ oder R³-COO-(CH₂CH(OOR⁴)CH₂)-OH auf. Glycerinmonofettsäureester weisen die Formel R³-COO-(CH₂CH(OH)CH₂)-OH oder HO-(CH₂CH(OOR³)CH₂)-OH auf. Dabei sind R³ und R⁴ unabhängig voneinander aus C₆-C₂₈ Alkyl und C₆-C₂₈ Alkenyl ausgewählt. Glycerinmonofettsäureester enthalten eine Glycerin- Gruppe, welche über eine Esterbindung an eine einzige Fettsäure gebunden. Beispiele sind Glycerinmonostearat, Glycerinmonobehenat, Glycerinmonocaprylat, Glycerinmonocaprat und Glycerinmonolaurat.

Polyoxyethylenether sind Verbindungen der Formel R⁵(OC2H3)ₙOH, wobei *R⁵* ausgewählt ist aus C6-C28 Alkyl, C6-C28 Alkenyl, substituierte und unsubstituierte Phenoxy-Gruppen; und n eine ganze Zahl größer 1 ist. Vorzugsweise wird der Polyoxyethylenether eines oder mehrerer Fettalkohole aus der Gruppe Steareth-2, Steareth-21, Makrogol-Cetostearylether 12, Ceteareth-25, Makrogol-Cetostearylether 20 und Mischungen der vorgenannten Verbindungen ausgewählt. Noch bevorzugter ist der Polyoxyethylenether eine Verbindung ausgewählt aus der Gruppe von Ceteareth-25, Makrogol-Cetostearylether 20 und Mischungen der vorgenannten Verbindungen.

Der Begriff "Polyglycerinether von Fettalkoholen" bezieht sich auf eine Verbindung der Formel R⁶O-(C3H6O2)n-H, wobei R⁶ ein verzweigtes oder lineares C6-C28 Alkyl oder C6-C28 Alkenyl ist und n eine ganze Zahl größer als 1, vorzugsweise eine ganze Zahl von 2 bis 10, ist. Es wird bevorzugt, dass die Zusammensetzung 0,01 bis 15,0 Gew.-%, 0,1 bis 10,0 Gew.-% oder 1 bis 5,0 Gew.-% Polyglycerinether enthält.

Der Begriff "Polyglycerinester von Fettsäuren" bezieht sich auf Verbindungen, die sowohl eine Polyglycerineinheit als auch mindestens eine C6-C26 Alkyl- oder C6-C26 Alkenylcarbonsäureeinheit enthalten. Diese Verbindungen können die Formel R⁷-R⁸-(C3H6O2)n-H aufweisen, wobei R⁷ ein C6-C26 Alkanoat- oder C6-C26 Alkenoat-Rest ist und R⁸ ein geeignetes Verbindungsmolekül oder eine direkte Bindung ist. Somit können die Polyglycerineinheit und die C6-C26 Alkyl- oder C6-C26 Alkenylcarbonsäureeinheit direkt durch eine Esterbindung verbunden sein oder eine Verbindungseinheit enthalten, die diese beiden Einheiten miteinander verbindet. Nicht einschränkende Beispiele für diese Gruppe sind Polyglyceryl-3-methylglucosedistearat, Polyglycerinpolycrinoleat, Polyglyceryl- Dimerat-Isostearat, Polyglyceryl-2-Laurat, Polyglyceryl-2-Sesquiisostearat, Polyglyceryl- 3-Distearat (Cremophor GS 32), Polyglyceryl-3-Oleat, Polyglyceryl-3-Methylglykose- Distearat, Polyglyceryl-4-Caprat (Polyglycerolcaprat T2010190), Polyglyceryl-4- Diisostearat / Polyhydroxystearat / Sebacat (Isolan GPS) und Polyglyceryl-4-Isostearat.

In einer Ausführungsform umfasst das Tensid ein kationisches Tensid, wie beispielsweise ein quaternäres Tensid. Quaternäre Tenside enthalten mindestens ein N-Atom, das mit 4 Alkyl- oder Arylgruppen kovalent verbunden ist. Dies führt, unabhängig vom pH-Wert, zu einer positiven Ladung. Vorteilhaft sind Alkylbetain, Alkylamidopropylbetain und Alkylamidopropylhydroxysulfain. Die erfindungsgemäß verwendeten kationischen Tenside können ferner bevorzugt gewählt werden aus der Gruppe der quaternären Ammoniumverbindungen, insbesondere Benzyltrialkylammoniumchloride oder -bromide, wie beispielsweise Benzyldimethylstearylammoniumchlorid, ferner Alkyltrialkylammoniumsalze, beispielsweise Cetyltrimethylammoniumchlorid oder - bromid, Alkyldimethylhydroxyethylammoniumchloride oder -bromide, Dialkyldimethylammoniumchloride oder -bromide, Alkylamidethyltrimethylammoniumethersulfate, Alkylpyridiniumsalze, beispielsweise Lauryl- oder Cetylpyrimidiniumchlorid, Imidazolinderivate und Verbindungen mit kationischem Charakter wie Aminoxide, beispielsweise Alkyldimethylaminoxide oder Alkylaminoethyldimethylaminoxide. Vorteilhaft sind insbesondere Cetyltrimethylammoniumsalze zu verwenden.

In einer weiteren Ausführungsform enthält die erfindungsgemäß verwendete Zusammensetzung mindestens ein Additiv. Bevorzugt sind Additive, die üblicherweise in Shampoos, Conditionern, Emulsionen, Haarwässern und einem Tonikum zur Behandlung der Kopfhaut und der Haare eingesetzt werden. Das mindestens eine Additiv kann in einem Anteil von 0,01 Gew.-% bis 12,0 Gew.-%, mehr bevorzugt von 0,25 Gew.-% bis 10,0 Gew.-%, insbesondere von 1,0 bis 7,0 Gew.-% vorliegen.

Das mindestens eine Additiv kann ferner ausgewählt sein aus der Gruppe bestehend aus Haarkonditionierungsmitteln, Rückfettern, Konservierungsmitteln, Stabilisatoren, Duftstoffen, Antioxidantien, Rheologiemodifikatoren, Verdickungsmitteln, Pflegemitteln, Farbstoffen, Perlganzmitteln, Aufhellungsmitteln, Lösungsmitteln und Kombinationen hiervon.

Haarkonditionierungsmittel können statische Aufladungen der Haare durch das Neutralisieren von elektrischer Ladung an ihrer Oberfläche verringern. Beispiele für Haarkonditionierungsmittel sind quartäre Ammoniumverbindungen.

Rückfetter, auch Rückfettungsmittel oder Überfettungsmittel genannt, sind lipophile Substanzen, die eine störende Auswirkung auf die epidermale Barrierefunktion verhindern können. Beispiele für Rückfetter sind Wollwachs, Squalen, flüssiges Paraffin, pflanzliche Öle, Silikone und Cetylpalmitat.

Konservierungsmittel sind Substanzen, die zur Konservierung verwendet werden, indem sie die Zusammensetzung zersetzende Mikroorganismen abtöten und/oder deren Wachstum hemmen. Vorzugsweise können die Konservierungsmittel ausgewählt sein aus der Gruppe bestehend aus Benzoesäure, Benzoesäurederivaten, Sorbinsäure, Sorbinsäurederivaten, Salicylsäure, Salicylsäurederivaten, Phenoxyethanol, Parabenen und Kombinationen hiervon. In einer bevorzugten Ausführungsform werden Natriumbenzoat und/oder Kaliumsorbat als Konservierungsmittel in der erfindungsgemäßen Zusammensetzung eingesetzt. Natriumbenzoat setzt in leicht saurem Milieu Benzoesäure und Kaliumsorbat Sorbinsäure frei. Beiden Säuren wird eine antimikrobielle Wirkung zugeschrieben.

Stabilisatoren können lichtempfindliche Komponenten gegenüber Strahlung schützen und sind bevorzugt UV-Absorber wie beispielsweise Benzophenonderivate.

Die Zugabe von Duftstoffen kann für einen angenehmen Geruch der Zusammensetzung sorgen. Beispiele sind die dem Fachmann bekannten Parfums.

Ein Antioxidans oder Antioxidationsmittel ist eine chemische Verbindung, die eine Oxidation anderer Komponenten in der erfindungsgemäßen Zusammensetzung verlangsamt oder gänzlich verhindert. Als Antioxidantien kommen beispielsweise Zitronensäure, Ascorbinsäure und Butylhydroxyanisol in Frage.

Rheologiemodifikatoren und Verdickungsmittel können dabei helfen, die Applikationseigenschaften der erfindungsgemäßen Zusammensetzung zu verbessern. Als Rheologiemodifikator und Verdickungsmittel kann die Zugabe von Kochsalz (Natriumchlorid) in Betracht gezogen werden. Durch die Zugabe von Kochsalz kann die Fließfähigkeit der erfindungsgemäßen Zusammensetzung in gewissen Grenzen beeinflusst und auf das nötige Maß eingestellt werden. Als Verdicker können zusätzlich Cellulosederivate oder Polyacrylate eingesetzt werden.

Im Rahmen der Anmeldung sollen unter Pflegemitteln Substanzen verstanden werden, welche die Haare und/oder die Kopfhaut pflegen. Hydrolysiertes Weizenprotein und Allantoin wirken pflegend auf Kopfhaut und Haare. Hydrolysiertes Weizenprotein hat hauptsächlich feuchtigkeitsspendende Eigenschaften.

Farbstoffe werden optional dazu verwendet, der erfindungsgemäßen Zusammensetzung eine charakteristische Farbe zu verleihen, so dass diese leicht von anderen Produkten unterschieden werden kann. Im Rahmen der Erfindung können Farbstoffe jedoch auch dazu verwendet werden, eine Färbung des menschlichen Haares herbeizuführen.

Als Lösungsmittel kann ein für den Fachmann auf diesem Gebiet gängiges Lösungsmittel oder Lösungsmittelgemisch eingesetzt werden. Bevorzugte Lösungsmittel sind Ethanol oder Butylenglykol, insbesondere 1,4-Butylenglykol, Propylenglykol und Isopropylalkohol. Bevorzugt ist Ethanol. Diese Lösungsmittel können vorzugsweise in einer Menge von 0,1 bis 70 Gew.% in der erfindungsgemäßen Zusammensetzung enthalten sein. Lösungsmittel können vorzugsweise in Zusammensetzungen, die bei ihrer Anwendung auf dem Kopf verbleiben (Leave-On-Formulierungen), enthalten sein. Ihre Verwendung kann zu einem Frischegefühl führen und belastet durch das schnelle Abtrocknen das Frisurenbild in einem sehr geringen Maß. Außerdem helfen Lösungsmittel bei der Penetration der Wirkstoffe und verstärken somit deren Wirksamkeit.

In einer bevorzugten Ausführungsform (nämlich als Tonikum/Haarwasser) enthält die topische Zusammensetzung Ethanol bevorzugt im Bereich von 20 bis 80 Gew.%, bezogen auf das Gesamtgewicht der Zusammensetzung. Bevorzugt enthält sie Ethanol in einer Menge von 30 bis 60 Gew.% und besonders bevorzugt von 35 bis 45 Gew.%, jeweils bezogen auf das Gesamtgewicht der Zusammensetzung. In diesen Bereichen unterstützt Ethanol die Penetration der Wirkstoffe deutlich, insbesondere die von Dimethylglycin und/oder seinen Salzen und auch die von Koffein und die anderer optional enthaltender Wirkstoffe, und verstärkt somit deren Wirksamkeit. Schließlich vermittelt Ethanol ein Frischegefühl und belastet durch das schnelle Abtrocknen das Frisurenbild in einem sehr geringen Masse.

Erfindungsgemäß wird die Zusammensetzung topisch angewendet. Unter einer topischen Anwendung ist eine äußerliche Anwendung, insbesondere eine örtliche, äußerliche Anwendung zu verstehen. Erfindungsgemäße Zusammensetzungen sind bevorzugt Kopfhaut- und Haarpflegeprodukte (d.h. Behandlungsmittel wie Shampoos, Conditioner, Kuren, Emulsionen, Lotionen, Duschbäder, und/oder ein Tonikum/Haarwasser) und keine (reinen) Styling-Produkte. Mit anderen Worten steht bei den topischen Zusammensetzungen der Erfindung der Zweck des Haar-Stylings, bei dem die Wirkung auf dem Haar selbst und nicht auf der Kopfhaut entfaltet wird, gerade nicht im Vordergrund. Vielmehr liegt der Fokus auf der medizinischen/pharmazeutischen Behandlung der Kopfhaut und der Haare.

In einer bevorzugten Ausführungsform sind die Bestandteile, insbesondere der Wirkstoff Dimethylglycin bzw. die Salze von Dimethylglycin wie beansprucht und die anderen Wirkstoffe zur Behandlung der Kopfhaut und der Haare, sofern vorhanden, in der erfindungsgemäßen Zusammensetzung homogen in einer wässrigen Phase verteilt. Der Begriff "wässrige Phase" schließt dabei die mögliche Anwesenheit von mit Wasser mischbaren organischen Lösungsmitteln (z.B. Alkohole) ein. Das bedeutet insbesondere, dass die erfindungsgemäß verwendete Zusammensetzung bevorzugt keine Trägersubstanzen aufweist. Bevorzugt enthält die erfindungsgemäß verwendete Zusammensetzung keine lamellare Struktur (insbesondere keine Vesikel und/oder lamellare Doppelmembranstruktur und/oder Liposome). Sie enthält bevorzugt auch keine lamellaren Strukturen (insbesondere Vesikel und/oder lamellare Doppelmembranstrukturen) ausbildende Substanz(en).

In einer bevorzugten Ausführungsform kann die erfindungsgemäß verwendete Zusammensetzung in Form einer Leave-On-Formulierung oder Rinse-Off- Formulierung vorliegen.

Eine Leave-On-Formulierung zeichnet sich dadurch aus, dass sie nach der Anwendung in Kontakt mit der zu behandelnden Kopfhaut und/oder den zu behandelnden Haaren bleibt. Hierdurch wird ein Art Wirkstoffdepot erzeugt, welches seine Wirksamkeit über einen längeren Zeitraum entfalten kann. Leave-On-Formulierungen können beispielsweise als Hydrogel oder Emulsion formuliert werden oder als wässrige oder wässrig-alkoholische Lösung (Tonikum) vorliegen. Vorzugsweise liegen Leave-On- Formulierungen als leicht viskose Formulierungen vor, sodass sie konzentriert auf der Kopfhaut und/oder den Haaren verbleiben und weniger stark in den Haarschaft spreiten können. Allerdings ist bei diesen Leave-On-Formulierungen zu beachten, dass die Haare durch die Konsistenz gebenden Stoffe nicht belastet werden, da diese dann einen etwas ungepflegten Eindruck hinterlassen.

Eine Rinse-Off-Formulierung zeichnet sich dadurch aus, dass nach der Anwendung die aufgetragenen Komponenten der erfindungsgemäß verwendeten Zusammensetzung wieder ausgespült werden. Auf diese Weise kann gegebenenfalls eine zu starke Belastung von Kopfhaut und/oder Haaren vermieden werden. Vorzugsweise können Rinse-Off-Formulierungen in Form eines Shampoos, Conditioners oder einer Kopfhaut- und/oder Haarkur vorliegen. In einer bevorzugten Ausführungsform sollte bei einer erfindungsgemäßen Zusammensetzung, die als Rinse-Off Formulierung angewendet wird, eine Einwirkzeit von ca. 2-5 min vorgesehen werden. Auf diese Weise kann die notwendige Penetration der Wirkstoffe in die Kopfhaut und/oder in die Haarwurzeln ermöglicht werden.

Die Erfindung betrifft die Behandlung von Haaren und der Kopfhaut die Behandlung und/oder Vorbeugung von Haarausfall. Dabei umfasst der Begriff Haarausfall Alopecia areata (kreisrunden Haarausfall), Alopecia androgenetica (erblich bedingten Haarausfall) bei Frauen und Männern, diffuse Alopezie (diffusen Haarausfall), altersbedingten Haarausfall (senescent Alopecia) und durch Chemotherapie-bedingten Haarausfall.

Besonders bevorzugt ist der zu behandelnde Haarausfall erblich bedingter Haarausfall (Alopecia androgenetica).

Ebenso besonders bevorzugt ist der zu behandelnde Haarausfall altersbedingter Haarausfall (senescent Alopecia).

In einer bevorzugten Ausführungsform kann die Verwendung eine kosmetische oder medizinische Verwendung sein.

Anhand der nachfolgenden Zusammensetzungen soll die Erfindung verdeutlicht werden, ohne sie jedoch auf die speziellen Beispiele einschränken zu wollen.

### Experimenteller Teil

Die folgenden Zusammensetzungen wurden durch für den Fachmann bekannte Homogenisierungsmaßnahmen hergestellt. Die Menge der Komponenten wurde jeweils so gewählt, dass ihr Gewichtsanteil in der fertigen Zusammensetzung angegebenen Gewichtsanteilen entspricht.

### Beispiel 1

Zusammensetzung in Form eines Shampoos (pH 4,8), welches die folgenden Komponenten enthält:

| | |
|---|---|
| Natriummyrethsulfat | 4,0 Gew.% |
| Natriumlaurethsulfat | 4,0 Gew.% |
| Dinatriumlaurethsulfosuccinat | 3,0 Gew.% |
| Tocopherylacetat | 0,3 Gew.% |
| Zitronensäure | 0,2 Gew.% |
| Koffein | 1,0 Gew.% |
| Na-Dimethylglycinat | 1,0 Gew.% |
| Parfum | 0,3 Gew.% |
| Kaliumsorbat | 0,2 Gew.% |
| Natriumbenzoat | 0,1 Gew.% |
| Wasser | ad 100 Gew.% |

### Beispiel 1a

Zusammensetzung in Form eines Shampoos (pH 4,9), welches die folgenden Komponenten enthält:

| | |
|---|---|
| Natriummyrethsulfat | 4,0 Gew.% |
| Natriumlaurethsulfat | 4,0 Gew.% |
| Dinatriumlaurethsulfosuccinat | 3,0 Gew.% |
| Tocopherylacetat | 0,3 Gew.% |
| Zitronensäure | 0,2 Gew.% |
| Na-Dimethylglycinat | 1,0 Gew.% |
| Parfum | 0,3 Gew.% |
| Kaliumsorbat | 0,2 Gew.% |
| Natriumbenzoat | 0,1 Gew.% |
| Wasser | ad 100 Gew.% |

### Beispiel 2

Zusammensetzung in Form eines Shampoos (pH 5,3), welches die folgenden Komponenten enthält:

| | |
|---|---|
| Natriummyrethsulfat | 4,0 Gew.% |
| Natriumlaurethsulfat | 4,0 Gew.% |
| Dinatriumlaurethsulfosuccinat | 3,0 Gew.% |
| Tocopherylacetat | 0,3 Gew.% |
| Zitronensäure | 0,2 Gew.% |
| Dimethylglycine HCl | 1,0 Gew.% |
| Parfum | 0,3 Gew.% |
| Kaliumsorbat | 0,2 Gew.% |
| Natriumbenzoat | 0,1 Gew.% |
| Wasser | ad 100 Gew.% |

### Beispiel 3

Zusammensetzung in Form eines Conditioners (pH 4,4), welches die folgenden Komponenten enthält:

| | |
|---|---|
| Natriumlaureth-11-carboxylat | 1,0 Gew.% |
| Polyquaternium-4/Hydroxypropylstärke Copolymer | 2,0 Gew.% |
| Steareth-8 | 5,0 Gew.% |
| Cetylalkohol | 2,0 Gew.% |
| Parfum | 0,5 Gew.% |
| Na-Dimethylglycinat | 0,4 Gew.% |
| Hydrolysiertes Kreatin | 0,5 Gew.% |
| Zitronensäure | 0,2 Gew.% |
| Natriumbenzoat | 0,05 Gew.% |
| Kaliumsorbat | 0,15 Gew.% |
| Wasser | ad 100 Gew.% |

### Beispiel 4

Zusammensetzung in Form einer Emulsion (pH 5,5), welche die folgenden Komponenten enthält:

| | |
|---|---|
| PEG-40 (hydriertes Rizinusöl) | 6,0 Gew.% |
| Cetearylalkohol | 0,3 Gew.% |
| Cera Alba | 0,7 Gew.% |
| Myristylmyristat | 1,0 Gew.% |
| Hexyldecanol | 5,0 Gew.% |
| Dicaprylether | 4,0 Gew.% |
| Dioctylclyclohexan | 3,0 Gew. % |
| Tocopherylacetat | 1,0 Gew.% |
| Octylmethoxycinnamat | 2,0 Gew.% |
| 4-Isobutyl-dibenzoylmethan | 1,0 Gew.% |
| Ascorbylpalmitat | 0,2 Gew. % |
| Retinylpalmitat | 0,05 Gew.% |
| 1,4-Butylenglykol | 4,0 Gew.% |
| Carbomer | 0,3 Gew. % |
| Hexyldecanol & Hexyldecyllaurat | 1,0 Gew.% |
| Zitronensäure | (pH 5,5) q.s. |
| Na-Dimethylglycinat | 0,4 Gew.% |
| Parfum | 0,2 Gew.% |
| Wasser | ad 100 Gew.% |

### Beispiel 5

Zusammensetzung in Form einer Öl-in-Wasser-Emulsion (pH 4,0), welche die folgenden Komponenten enthält:

| | |
|---|---|
| Glycerinmonostearat | 2 Gew.% |
| Cetylalkohol | 3 Gew.% |
| Cetylphosphat | 0,4 Gew.% |
| Paraffinöl, subliquidum | 15 Gew.% |
| Vaseline | 3 Gew.% |
| Caprylcaprinsäuretriglycerid | 4 Gew.% |
| Octyldodecanol | 2 Gew.% |
| hydriertes Kokosfett | 2 Gew.% |
| Glycerin | 3 Gew.% |
| Glykolsäure | (pH 4,0) q.s. |
| Na-Dimethylglycinat | 0,4 Gew.% |
| Parfümöl | q.s. |
| Konservierungsmittel | q.s. |
| Wasser | ad 100 Gew.% |

### Beispiel 6

Zusammensetzung in Form einer Wasser-in-Öl Emulsion (pH 5,9), welche die folgenden Komponenten enthält:

| | |
|---|---|
| PEG-7-hydriertes Ricinusöl | 4 Gew.% |
| Wollwachsalkohol | 1,5 Gew.% |
| Bienenwachs | 3 Gew.% |
| Triglycerid, flüssig | 5 Gew.% |
| Vaseline | 9 Gew.% |
| Stearylalkohol | 4 Gew.% |
| Paraffinöl, subliquidum | 4 Gew.% |
| Glycerin | 2 Gew.% |
| Magnesiumsulfat 7 H2O | 0,7 Gew.% |
| Na-Dimethylglycinat | 0,4 Gew.% |
| Milchsäure | (pH 5,9) q.s. |
| Parfümöl | q.s. |
| Konservierungsmittel | q.s. |
| Wasser | ad 100 Gew.% |

### Beispiel 7

Zusammensetzung in Form eines Shampoos (pH 5,0), welches die folgenden Komponenten enthält:

| | |
|---|---|
| Natriumlaurethsulfat | 12,0 Gew.% |
| Cocoamidopropylbetain | 3,5 Gew.% |
| Disodiumcocoamphodiacetat | 3,0 Gew.% |
| Zitronensäure | 0,2 Gew.% |
| Na-Dimethylglycinat | 1,0 Gew.% |
| Parfum | 0,3 Gew.% |
| Kaliumsorbat | 0,2 Gew.% |
| Natriumbenzoat | 0,1 Gew.% |
| Wasser | ad 100 Gew.% |

### Referenzbeispiel 1

Zusammensetzung in Form eines Shampoos (ohne Na-Dimethylglycinat; pH 5,1), welches die folgenden Komponenten enthält:

| | |
|---|---|
| Natriummyrethsulfat | 4,0 Gew.% |
| Natriumlaurethsulfat | 4,0 Gew.% |
| Dinatriumlaurethsulfosuccinat | 3,0 Gew.% |
| Tocopherylacetat | 0,3 Gew.% |
| Zitronensäure | 0,2 Gew.% |
| Parfum | 0,3 Gew.% |
| Kaliumsorbat | 0,2 Gew.% |
| Natriumbenzoat | 0,1 Gew.% |
| Wasser | ad 100 Gew.% |

### Referenzbeispiel 2

Zusammensetzung in Form einer Öl-in-Wasser-Emulsion (pH 4,0), welche die folgenden Komponenten enthält:

| | |
|---|---|
| Glycerinmonostearat | 2 Gew.% |
| Cetylalkohol | 3 Gew.% |
| Cetylphosphat | 0,4 Gew.% |
| Paraffinöl, subliquidum | 15 Gew.% |
| Vaseline | 3 Gew.% |
| Caprylcaprinsäuretriglycerid | 4 Gew.% |
| Octyldodecanol | 2 Gew.% |
| hydriertes Kokosfett | 2 Gew.% |
| Glycerin | 3 Gew.% |
| Glykolsäure | (pH 4,0) q.s. |
| Parfümöl | q.s. |
| Konservierungsmittel | q.s. |
| Wasser | ad 100 Gew.% |

### Referenzbeispiel 3

Zusammensetzung in Form einer Öl-in-Wasser-Emulsion (pH 8,0), welche die folgenden Komponenten enthält:

| | |
|---|---|
| Glycerinmonostearat | 2 Gew.% |
| Cetylalkohol | 3 Gew.% |
| Cetylphosphat | 0,4 Gew.% |
| Paraffinöl, subliquidum | 15 Gew.% |
| Vaseline | 3 Gew.% |
| Caprylcaprinsäuretriglycerid | 4 Gew.% |
| Octyldodecanol | 2 Gew.% |
| hydriertes Kokosfett | 2 Gew.% |
| Glycerin | 3 Gew.% |
| Natriumhydroxid | (pH 8,0) q.s. |
| Na-Dimethylglycinat | 0,4 Gew.% |
| Parfümöl | q.s. |
| Konservierungsmittel | q.s. |
| Wasser | ad 100 Gew.% |

### Beispiel 8

Zusammensetzung in Form eines Haarwassers (pH 5,9), welches die folgenden Komponenten enthält:

| | |
|---|---|
| Alkohol denat. (Ethanol) | 30,0 Gew.% |
| Amodimethicon | 0,5 Gew.% |
| Panthenol | 0,5 Gew.% |
| Koffein | 0,5 Gew.% |
| Na-Dimethylglycinat | 0,5 Gew.% |
| Bisabolol | 0,2 Gew.% |
| PEG-25 PABA | 0,5 Gew.% |
| PEG-16 (hydriertes Rizinusöl) | 0,5 Gew.% |
| Parfum | 0,3 Gew.% |
| Menthol | 0,3 Gew.% |
| Milchsäure | 0,1 Gew.% |
| Wasser | ad 100 Gew.% |

### Beispiel 8a

Zusammensetzung in Form eines Haarwassers (pH 5,5), welches die folgenden Komponenten enthält:

| | |
|---|---|
| Alkohol denat. (Ethanol) | 30,0 Gew.% |
| Amodimethicon | 0,5 Gew.% |
| Panthenol | 0,5 Gew.% |
| Na-Dimethylglycinat | 0,5 Gew.% |
| Bisabolol | 0,2 Gew.% |
| PEG-25 PABA | 0,5 Gew.% |
| PEG-16 (hydriertes Rizinusöl) | 0,5 Gew.% |
| Parfum | 0,3 Gew.% |
| Menthol | 0,3 Gew.% |
| Milchsäure | 0,1 Gew.% |
| Wasser | ad 100 Gew.% |

### Beispiel 9

Zusammensetzung in Form eines Haarwassers (pH 5,5), welches die folgenden Komponenten enthält:

| | |
|---|---|
| Alkohol denat. (Ethanol) | 30,0 Gew.% |
| Amodimethicon | 0,5 Gew.% |
| Panthenol | 0,5 Gew.% |
| Dimethylglycine HCl | 0,5 Gew.% |
| Bisabolol | 0,2 Gew.% |
| PEG-25 PABA | 0,5 Gew.% |
| PEG-16 (hydriertes Rizinusöl) | 0,5 Gew.% |
| Parfum | 0,3 Gew.% |
| Menthol | 0,3 Gew.% |
| Milchsäure | 0,1 Gew.% |
| Wasser | ad 100 Gew.% |

### Referenzbeispiel 4

Zusammensetzung in Form eines Haarwassers, welches die folgenden Komponenten enthält:

| | |
|---|---|
| .Alkohol denat. (Ethanol) | 30,0 Gew.% |
| Amodimethicon | 0,5 Gew.% |
| Panthenol | 0,5 Gew.% |
| Bisabolol | 0,2 Gew.% |
| PEG-25 PABA | 0,5 Gew.% |
| PEG-16 (hydriertes Rizinusöl) | 0,5 Gew.% |
| Parfum | 0,3 Gew.% |
| Menthol | 0,3 Gew.% |
| Milchsäure | 0,1 Gew.% |
| Wasser | ad 100 Gew.% |

### Referenzbeispiel 5

Zusammensetzung in Form eines Haarwassers, welches die folgenden Komponenten enthält:

| | |
|---|---|
| Alkohol denat. (Ethanol) | 30,0 Gew.% |
| Amodimethicon | 0,5 Gew.% |
| Panthenol | 0,5 Gew.% |
| Bisabolol | 0,2 Gew.% |
| PEG-25 PABA | 0,5 Gew.% |
| PEG-16 (hydriertes Rizinusöl) | 0,5 Gew.% |
| Parfum | 0,3 Gew.% |
| Menthol | 0,3 Gew.% |
| Milchsäure | 0,1 Gew.% |
| Wasser | ad 100 Gew.% |

### Studiendesign und -ergebnisse

1. Im Rahmen einer in vitro Untersuchung wurde die Wirkung von Na-Dimethylglycinat im Zellkulturmodell mit humanen hornbildenden Keratinozyten- Zellen untersucht. Hierfür wurden HaCaT-Zellen für 1, 3, 5, und 7 Tage in DMEM- Medium (inklusive fötalem Kälberserum und einem Antibiotika-Antimykotika-Mix) kultiviert und u.a. die Viabilität und Proliferation der Zellen durch geeignete Messmethoden bestimmt sowie die Expression der für das Zellwachstum relevanten Wachstumsfaktoren bestimmt.

### Nachweis der Viabilität:

Für diese Messung wurde ein sogenannter MTT-Assay verwendet, um die zelluläre Stoffwechselaktivität als Indikator für die Lebensfähigkeit und Zytotoxizität der Zellen zu bestimmen. Dieser kolorimetrische Assay basiert auf der Reduktion eines gelben Tetrazoliumsalzes (3-(4,5-Dimethylthiazol-2-yl)-2,5-diphenyltetrazoliumbromid oder MTT) zu violetten Formazan-Kristallen durch metabolisch aktive Zellen.

Humane epidermale Keratinozyten-Zellen (HaCaT) wurden in einer 96-Well Platte mit einer Zelldichte von 5.000 Zellen/Well ausgesät und in Medium (DMEM mit 10% FBS, 1% Penicillin-Streptomycin, 0.5% Fungizone) kultiviert. Am nächsten Tag sowie nach weiteren 24 h sowie 48 h und 72 h Kultivierung bei 37°C und 5 Vol.-% CO2 wurde das Zellkulturmedium ohne (Kontrolle) bzw. mit den jeweils bereits enthaltenen Wirkstoff-Konzentrationen von Natrium-Dimethylglycinate (DMG) gewechselt. Die Messung erfolgte analog zu bereits publizierten Untersuchungen in B. I. Tóth, N. Dobrosi, A. Dajnoki, G. Czifra, A. Oläh, A. G. Szöllösi, I. Juhäsz, K. Sugawara, R. Paus, T. Bíró, J Invest Dermatol 2011, 131, 1095-1104.

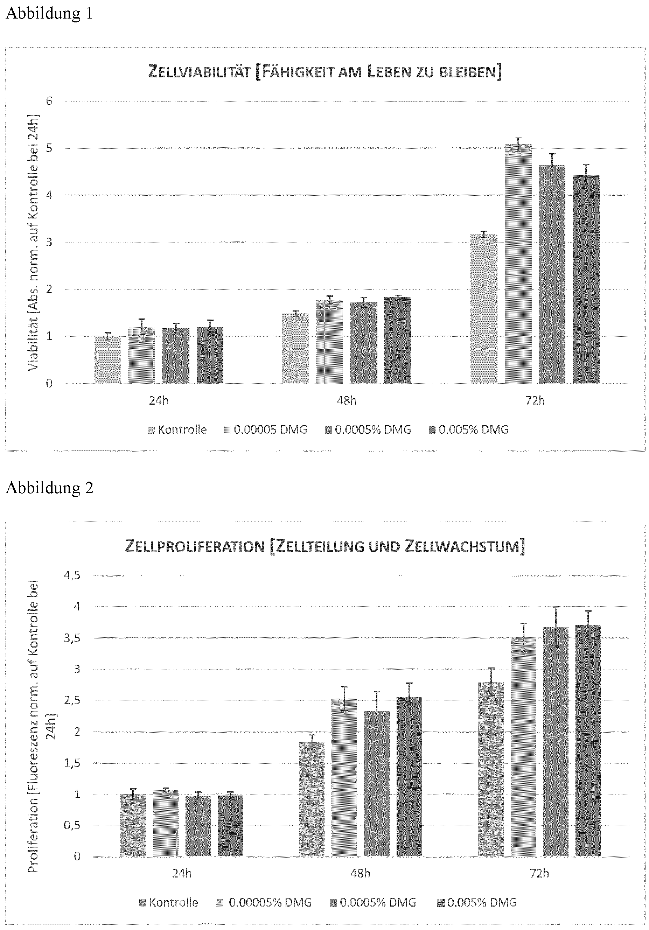

In Abbildung 1 ist die Zellviabilität nach 24 h, 48 h und 72 h Kultivierung dargestellt und eine deutliche Steigerung durch die Zugabe von DMG nach 48 h und 72 h zu sehen. Die Zellviabilität wurde bestimmt mittels MTT-Assay. Die Absorption wurde jeweils in Vierfachbestimmung gemessen und auf die Kontrolle bei 24 h normiert. Angegeben ist der Mittelwert mit Standardabweichung.

### Nachweis der Proliferation:

Für die Messung der Proliferation wurde ein sogenannter CyQUANT-Assay durchgeführt. Bei diesem fluoreszenz-basierten Assay bindet der verwendete Fluoreszenzfarbstoff an DNA (engl. deoxyribonucleic acid), wobei der Gehalt an zellulärer DNA ein direktes Maß für die Anzahl an Zellen innerhalb einer Probe ist.

Humane epidermale Keratinozyten-Zellen (HaCaT) wurden in einer 96-Well Platte mit einer Zelldichte von 5.000 Zellen/Well ausgesät und in Medium (DMEM mit 10% FBS, 1% Penicillin-Streptomycin, 0.5% Fungizone) kultiviert. Am nächsten Tag sowie nach weiteren 24 h sowie 48 h und 72 h Kultivierung bei 37°C und 5 Vol.-% CO2 wurde das Zellkulturmedium ohne (Kontrolle) bzw. mit den jeweils bereits enthaltenen Wirkstoff-Konzentrationen gewechselt. Die Messung erfolgte analog zu bereits publizierten Untersuchungen in A. Oläh, B. I. Tóth, I. Borbíró, K. Sugawara, A. G. Szöllösi, G. Czifra, B. Päl, L. Ambrus, J. Kloepper, E. Camera, The Journal of clinical investigation 2014, 124, 3713-3724.

In Abbildung 2 ist die Zellproliferation nach 24 h, 48 h und 72 h Kultivierung dargestellt und eine deutliche Steigerung durch die Zugabe von DMG nach 48 h und 72 h zu sehen. Die Zellproliferation wurde bestimmt mittels CyQUANT-Assay. Die Fluoreszenz wurde jeweils in Dreifachbestimmung gemessen und auf die Kontrolle bei 24 h normiert. Angegeben ist der Mittelwert mit Standardabweichung.

### Nachweis der Genexpression von VEGF:

VEGF (engl. Vascular Endothelial Growth Factor) fördert das Wachstum und die Bildung neuer Blut- und Lymphgefäße. Die Messung der Genexpression erfolgte über ein StandardVerfahren der sogenannten quantitative Echtzeit-PCR (qRT-PCR).

Humane epidermale Keratinozyten-Zellen (HaCaT) wurden in einer 6-Well Platte mit einer Zelldichte von 140.000 Zellen/Well ausgesät und in Medium (DMEM mit 10% FBS, 1% Penicillin-Streptomycin, 0.5% Fungizone) bei 37°C und 5 Vol.-% CO2 kultiviert. Am nächsten Tag wurde das Zellkulturmedium ohne (Kontrolle) bzw. mit den jeweils bereits enthaltenen Wirkstoff-Konzentrationen gewechselt und die Zellen nach 24 h geerntet. Die Messung der Genexpression erfolgte mittels qRT-PCR basierend auf bereits publizierten Untersuchungen in B. V. Diaz, M.-C. Lenoir, A. Ladoux, C. Frelin, M. Démarchez, S. Michel, Journal of Biological Chemistry 2000, 275, 642-650.

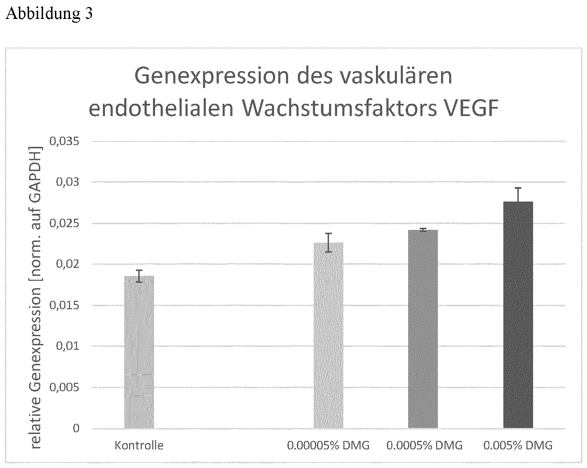

In Abbildung 3 ist die Genexpression von VEGF nach 24 h Kultivierung dargestellt und eine deutliche Steigerung der Genexpression durch die Zugabe von DMG zu sehen. Dargestellt ist die mittels qRT-PCR ermittelte relative Genexpression (Dreifachbestimmung, normiert auf die jeweilige Genexpression eines konstitutiv exprimiertes Gens; GAPDH - Glycerinaldehyd-3-phosphat- Dehydrogenase) von VEGF. Angegeben ist der Mittelwert mit Standardabweichung.

Es hat sich überraschend gezeigt, dass mit DMG die für das Wachstum relevanten Parameter der HaCaT-Zellen positiv beeinflußt wurden und die Expression des Wachstumsfaktors VEGF signifikant gesteigert wurde gegenüber der Behandlung von HaCaT-Zellen ohne DMG.

2. Das erfindungsgemäße Shampoo aus Beispiel 1 und das Shampoo aus Referenzbeispiel 1 wurden zudem im Rahmen einer Anwendungsstudie bei 30 Männern und 30 Frauen für 6 Monate angewendet. Die Probanden wurden zur subjektiven Bewertung des Rückgangs ihres Haarausfalls nach Beendigung der Anwendung befragt, wobei diese Ergebnisse mit Hilfe von Fragebögen festgehalten wurden. Signifikant weniger Haare pro Tag fielen bei Anwendung des erfindungsgemäßen Shampoos aus Beispiel 1 aus.

3. Das erfindungsgemäße Haarwasser aus Beispiel 8 und das Haarwasser aus Referenzbeispiel 4 wurden zudem im Rahmen einer Anwendungsstudie bei 30 Männern und 30 Frauen für 6 Monate angewendet. Die Probanden wurden zur subjektiven Bewertung des Rückgangs ihres Haarausfalls nach Beendigung der Anwendung befragt, wobei diese Ergebnisse mit Hilfe von Fragebögen festgehalten wurden.

Signifikant weniger Haare pro Tag fielen bei Anwendung des erfindungsgemäßen Haarwassers aus Beispiel 8 aus.

## Patentansprüche

1. Zusammensetzung, enthaltend Dimethylglycin gemäß Formel I und/oder ein Salz davon , 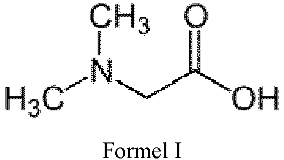
wobei das Salz von Dimethylglycin ein Alkali-, Erdalkali- oder Ammoniumsalz von Dimethylglycin oder das Salz einer anorganischen und/oder organischen Säure mit Dimethylglycin ist,
zur therapeutischen Verwendung in der topischen Behandlung von Haaren und der Kopfhaut, wobei die Behandlung der Haare und der Kopfhaut zur Behandlung und/oder Vorbeugung von Haarausfall erfolgt, wobei der Haarausfall ausgewählt ist aus Alopecia areata (kreisrundem Haarausfall), Alopecia androgenetica (erblich bedingter Haarausfall) bei Frauen und Männern, diffuser Alopezie (diffusem Haarausfall), altersbedingtem Haarausfall (senescent Alopecia) und durch Chemotherapie-bedingtem Haarausfall.

2. Zusammensetzung zur Verwendung gemäß Anspruch 1, wobei die Zusammensetzung eine Pflegezusammensetzung, bevorzugt zur Förderung des Stoffwechsels der Kopfhaut und der Haare, ist.

3. Zusammensetzung zur Verwendung gemäß einem der vorangehenden Ansprüche, wobei die Zusammensetzung einen pH-Wert im Bereich von 3,0 bis 5,9 aufweist.

4. Zusammensetzung zur Verwendung gemäß einem der vorangehenden Ansprüche, wobei die Zusammensetzung 0,00001 bis 25,0 Gew.% Dimethylglycin und/oder eines Salzes von Dimethylglycin gemäss Anspruch 1 enthält.

5. Zusammensetzung zur Verwendung gemäß einem der vorangehenden Ansprüche, wobei die Zusammensetzung Tenside in einer Menge von 2 bis 40 Gew.% enthält.

6. Zusammensetzung zur Verwendung gemäß einem der vorangehenden Ansprüche, wobei die Zusammensetzung mindestens einen weiteren Wirkstoff enthält, ausgewählt aus Koffein, Menthol, Biotin, Zink PCA, Niacinamid, Panthenol, Ectoin, Ubichinon-10; Taurin, Pantolacton, Echinacea, Carnitin, Tocopherylacetat und Kombinationen davon, und/oder
wobei die Zusammensetzung mindestens ein Additiv enthält, ausgewählt aus Haarkonditionierungsmitteln, Rückfettern, Konservierungsmitteln, Stabilisatoren,
Duftstoffen, Antioxidantien, Rheologiemodifikatoren, Verdickungsmitteln, Pflegemitteln, Farbstoffen, Perlganzmitteln, Aufhellungsmitteln, Lösungsmitteln und
Kombinationen hiervon.

## Claims

1. Composition, containing
dimethylglycine according to Formula I and/or a salt thereof, 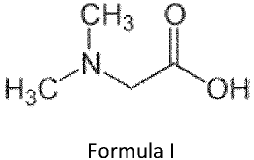 wherein the salt of dimethylglycine is an alkali, alkaline earth, or ammonium salt of dimethylglycine or the salt of an inorganic and/or organic acid with dimethylglycine, for therapeutic use in the topical treatment of hair and the scalp, wherein the treatment of hair and the scalp is for the treatment and/or prevention of hair loss, wherein the hair loss is selected from alopecia areata (circular hair loss), androgenetic alopecia (hereditary hair loss) in women and men, diffuse alopecia (diffuse hair loss), age-related hair loss (senescent alopecia), and chemotherapy-induced hair loss.

2. Composition for use according to claim 1, wherein the composition is a care composition, preferably for promoting the metabolism of the scalp and hair.

3. Composition for use according to any one of the preceding claims, wherein the composition has a pH value in the range of 3.0 to 5.9.

4. Composition for use according to any one of the preceding claims, wherein the composition contains 0.00001 to 25.0 wt.% dimethylglycine and/or a salt of dimethylglycine according to claim 1.

5. Composition for use according to any one of the preceding claims, wherein the composition contains surfactants in an amount of 2 to 40 wt.%.

6. Composition for use according to any one of the preceding claims, wherein the composition contains at least one further active ingredient selected from caffeine, menthol, biotin, zinc PCA, niacinamide, panthenol, ectoine, ubiquinone-10; taurine, pantolactone, echinacea, carnitine, tocopheryl acetate and combinations thereof, and/or wherein the composition contains at least one additive selected from hair conditioning agents, re-fattening agents, preservatives, stabilisers, fragrances, antioxidants, rheology modifiers, thickeners, care agents, dyes, pearlising agents, brighteners, solvents, and combinations thereof.

## Revendications

1. Composition contenant
de la diméthylglycine selon la formule I et/ou un sel de celle-ci, 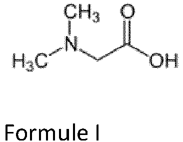
le sel de diméthylglycine étant un sel alcalin, alcalino-terreux ou d'ammonium de diméthylglycine ou le sel d'un acide inorganique et/ou organique avec la diméthylglycine, destinée à un usage thérapeutique dans le traitement topique des cheveux et du cuir chevelu, le traitement des cheveux et du cuir chevelu étant effectué pour traiter et/ou prévenir la chute des cheveux, la chute des cheveux étant choisie parmi l'alopécie areata (alopécie circulaire), l'alopécie androgénétique (alopécie héréditaire) chez les femmes et les hommes, l'alopécie diffuse (alopécie diffuse), l'alopécie liée à l'âge (alopécie sénescente) et l'alopécie liée à la chimiothérapie.

2. Composition pour l'utilisation selon la revendication 1, la composition étant une composition de soin, de préférence pour favoriser le métabolisme du cuir chevelu et des cheveux.

3. Composition pour l'utilisation selon l'une des revendications précédentes, la composition ayant un pH compris entre 3,0 et 5,9.

4. Composition pour l'utilisation selon l'une des revendications précédentes, la composition contenant 0,00001 à 25,0 % en poids de diméthylglycine et/ou d'un sel de diméthylglycine selon la revendication 1.

5. Composition pour l'utilisation selon l'une des revendications précédentes, la composition contenant des tensioactifs en une quantité comprise entre 2 et 40 % en poids.

6. Composition pour l'utilisation selon l'une des revendications précédentes, la composition contenant au moins un autre principe actif choisi parmi la caféine, le menthol, la biotine, le PCA de zinc, la niacinamide, le panthénol, l'ectoïne, l'ubiquinone-10, la taurine, le pantolactone, l'échinacée, la carnitine, l'acétate de tocophéryle et des combinaisons de ceux-ci, et/ou
dans laquelle la composition contient au moins un additif choisi parmi les agents revitalisants pour cheveux, les agents relipidants, les conservateurs, les stabilisants, les parfums, les antioxydants, les modificateurs de rhéologie, les épaississants, les agents d'entretien, les colorants, les agents perlants, les agents éclaircissants, les solvants et leurs combinaisons.
